# EUROPEAN PATENT APPLICATION

(11) **EP 3 900 733 A1**
(43) Date of publication of application: **27.10.2021**
(21) Application number: 20382337.2
(22) Date of filing: 24.04.2020
(51) Int. Cl.: A61K 38/08, A61K 45/00, A61P 17/02, A61P 17/14

(54) **SRC KINASE ACTIVATORS AND ENG FUNCTION INHIBITORS AS ENHANCERS OF SKIN HOMEOSTASIS/REGENERATION AND HAIR GROWTH**

(71) Applicant: Derma Innovate S.L., 14014 Córdoba (ES); Fundación para la Investigación Biomédica del Hospital Universitario Ramón y Cajal, 28034 Madrid (ES)
(72) Inventor: ESPADA REGALADO, Jesús, 28034 Madrid (ES); SANCHEZ, María Inmaculada, C.P.:28003 Madrid (ES); FERNÁNDEZ MARTOS, Sandra, 28034 Madrid (ES); MONTOYA MIÑANO, Juan José, C.P.:28003 Madrid (ES)
(74) Representative: Hoffmann Eitle

(57) **Abstract**

The present invention illustrates that a composition comprising a) a Src kinase activator and b) an Eng function inhibitor (iEng) is useful in a method of treatment which comprises the activation of a population of skin or epidermal stem cells and/or progenitor cells in a subject, preferably in a human subject.

## Description

### TECHNICAL FIELD

The invention relates to the use of Src kinase activators and Eng function inhibitors, optionally in combination with a photosensitive agent capable of producing reactive oxygen species (ROS), as enhancers of skin homeostasis/regeneration and hair growth.

### BACKGROUND OF THE INVENTION

The Photodynamic Therapy (PDT) is a therapeutic modality widely used for the treatment of various skin diseases, including cancer.

PDT is based on the exogenous administration of photosensitive compounds (PS), or precursors of the same, which accumulate by means of different mechanisms in the target tissues. The irradiation of the tissue with light of appropriate wavelength, usually in the red region of the spectrum (λ≧600 nm) for greater penetration into the tissue, and in the presence of intracellular oxygen, induces the production of Reactive Oxygen Species (ROS), especially singlet oxygen. The rapid accumulation of intracellular ROS above a critical threshold promotes a strong photosensitization that induces or leads to cell death.

5-aminolevulinic acid (ALA) and, to a greater extent, its methylated derivative methyl aminolevulinate (mALA), are two of the most widely used compounds in the clinical setting, in dermatological protocols with PDT. Their low molecular weight determines a high absorption through the epidermis allowing topical application of the same. These compounds are not photoactive by themselves, but act as precursors of endogenous PS (protoporphyrin IX (PpIX)). Once absorbed by the cell, these compounds are incorporated in the metabolic pathway of the biosynthesis of the heme group, thus promoting an abnormal accumulation of PpIX that can last from just a few hours to days, and lead to the photosensitization of the target tissue. The MAL-PDT treatment is widespread in clinical dermatology, particularly for the treatment of actinic keratosis and basal cell carcinoma.

It has been described that the experimental treatment with exogenous sources of ROS in low amounts, such as hydrogen peroxide, can promote cell proliferation in *in vitro* cultures (Boonstra J, Post J A. "Molecular events associated with reactive oxygen species and cell cycle progression in mammalian cells". Gene 337: 1-13, 2004), including potential neural progenitor cells grown by the system of neurospheres (Le Belle J E et al. "Proliferative neural stem cells have high endogenous ROS levels that regulate self-renewal and neurogenesis in a PI3K/Akt- dependant manner", Cell Stem Cell. 8: 59-71, 2011).

Moreover, there is now documental evidence (see US 20150335746 A1) suggesting a causal relationship between endogenous production of ROS in skin tissue and the functional activation of the stem cells contained therein, with potential clinical, pharmacologic or cosmetic use. In this sense, US 20150335746 A1 was the first to provide *in vivo* experimental procedures that allowed inducing a controlled endogenous production of ROS in skin tissue. In addition, US 20150335746 A1 provided experimental evidence indicating that endogenous ROS accumulation in tissues can be part of the normal skin homeostatic process functionally dependent on stem cells. In this sense, the inventors of US 20150335746 A1 described an experimental method that used PDT-MAL to induce the control endogenous production of ROS in the hair follicle that led to the activation of the epidermal stem cells contained in such niche. Such stimulation of the epidermal stem cells was due to the transcriptional activation by ROS in the target tissue of the genes of the prolactin family 2, also known as proliferins, particularly proliferin-2 or Pr12c3.

In the current art, one of the most widespread uses of the epidermal stem cells in the field of bioengineering is the generation of skin equivalents of epidermal or dermo-epidermal components (Shevchenko R V et al. "A review of tissue-engineered skin bioconstructs available for skin reconstruction", J. R. Soc. Interface 7: 229-58, 2010). These skin equivalents or artificial skins have very important applications in regenerative medicine, primarily for the treatment of burns and wounds of great extent and depth. The ideal treatment for this type of injury is the autograft with different types of skin equivalents generated from skin of the own patient. Given the restrictions in the European Union (Directive 2010/63/EU) and other countries for the use of experimental animals, another key application of skin equivalents is their use as biological models to test the feasibility and toxicity of pharmaceutical and cosmetic compounds. However, for these types of applications there is an essential limitation, that is the generation time of a functional skin equivalent, which requires the ex vivo expansion of the epidermal progenitors and the stratification of the epidermal component in contact with air. In a clinical setting, the excessively long time in the production of equivalents for autograft pose an immediate danger to the patient, which requires the use of alternative therapies such as cadaver skin grafts or little humanized synthetic equivalents. In pharmacology and cosmetics, the generation time of artificial skin is directly related to its production cost.

The present invention solves this problem by providing novel compositions and methods of use thereof capable of significantly speeding up these processes of formation of skin and hair equivalents.

On the other side and as already known, different experimental evidence indicates that many skin diseases, including different types of cancer, alopecia and processes such as aging, wrinkles etc.. may be due to a defect in the activity of epidermal stem cells, and in particular of those residing in the niche of the hair follicle. A major limitation in the clinical management of these diseases is that the treatments currently available are symptomatic and nonspecific, that is, they are not intended to functionally modulate the activity of the epidermal stem cells.

Therefore, a second problem that arises in the art is, therefore, the need to develop therapeutic and cosmetic methods more specific and effective than the current methods to treat diseases related to stem cells. The solution provided by the present invention provides a treatment method capable of significantly activating skin stem cells, in particular activating epidermal stem cells, in cells previously or simultaneously treated with photosensitive agents capable of producing reactive oxygen species (ROS), such as 5-aminolevulinic acid (ALA) or its methylated derivative methyl aminolevulinate (mALA).

### BRIEF DESCRIPTION OF THE INVENTION

The present invention generally refers to a pharmaceutical o cosmetic composition comprising a) a Src kinase activator **and** b) an Eng function inhibitor (iEng), preferably for use in a method of treatment which comprises the activation of a population of skin or epidermal stem cells and/or progenitor cells in a subject, preferably in a human subject. Preferably, the population of skin or epidermal stem cells and/or progenitor cells is a population of hair follicle stem cell and/or progenitor cells. More preferably, said method of treatment is performed subsequently or simultaneously to the treatment with a source of light such as a source of red light.

Other aspects and objectives of the present invention will become more apparent from the ensuring description, examples, and claims.

### BRIEF DESCRIPTION OF THE FIGURES

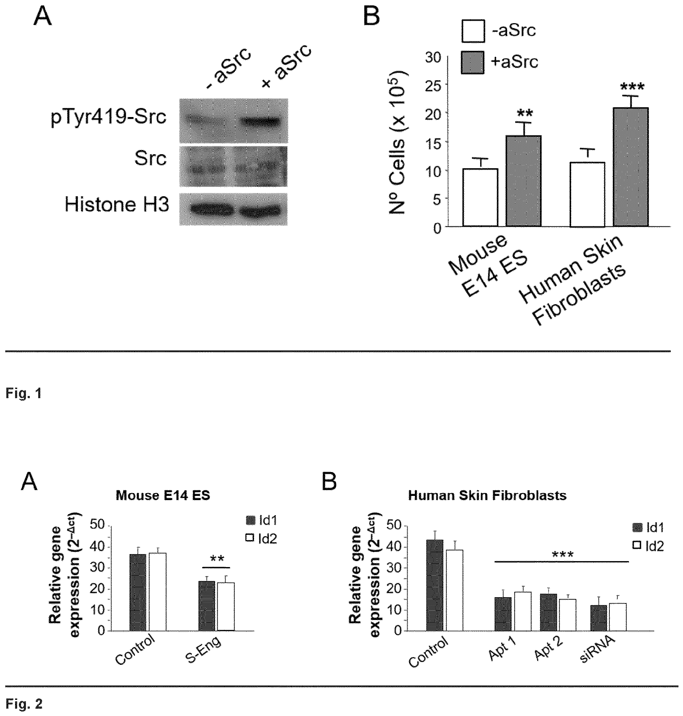

**Figure 1****.** A) Representative example showing Tyr419 cSRC phosphorilation induced by the YEEI phosphopeptide of SEQ ID NO 1 in mouse E14 embryonic stem cells. Similar results are observed in human skin fibroblasts. B) Representative examples showing the statistically significant induction of cell proliferation induced by the YEEI phosphopeptide of SEQ ID NO 1 in mouse E14 embryonic stem cells and human primary skin fibroblasts (48 hours after treatments). The mean +/- SEM is represented (n=3; **, p<0.05; *** p<0.001).
**Figure 2****.** A) Representative example of the operational inhibition of Endoglin function (iEng) in mouse E14 embryonic stem cells using the secreted/soluble form of Eng (S-Eng) of SEQ ID NO 5 acting as a ligand antagonist. B) Representative examples showing the significant inhibition of Eng function in human primary skin fibroblasts using different approaches, including treatments with aptamers (Apt 1 (SEQ ID NO 3) and Apt 2 (SEQ ID NO 4)) or a specific siRNA of SEQ ID NO 2 to inhibit the specific mRNA transcription. Endoglin function inhibition was evaluated by gene expression quantification (qRT-PCR) of two key targets, Id1 and Id2, of Eng signalling in stem cells, the skin and the hair follicle. The mean +/- SEM of three independent experiments is represented (**, p<0.05 ; *** p<0.001).

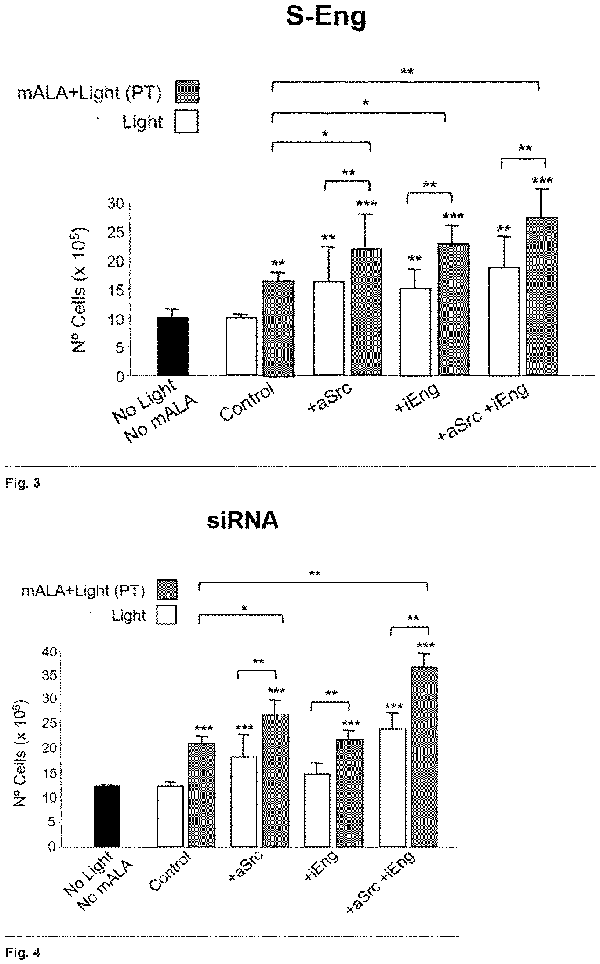

**Figure 3****.** Example showing the biological synergy of the activation of the cSrc kinase, using the YEEI phosphopeptide of SEQ ID NO 1, and the functional inhibition of Eng, using the soluble/secreted form of this protein of SEQ ID NO 5, in the context of a ROS-dependent stimulation of cell function. The basal activation of cell proliferation induced by the transient induction of ROS production, through phototreatments (PT) with mALA and red light, is significantly enhanced by a combination with activators of Src kinase function (aSrc; YEEI phosphopeptide (SEQ ID NO 1) and/or an Endoglin function inhibitor (iEng; a secreted/soluble form of Eng, S-Eng of SEQ ID NO 5), in mouse E14 embryonic stem cells. The results are measured, 48 hours after the irradiation treatment. The mean +/- SEM of three independent experiments is represented. Statistical significance is referred to Control, except when indicated (*, p<0.1; **, p<0.05 ; *** p<0.001).
**Figure 4****.** Example showing the biological synergy of the activation of the cSrc kinase, using the YEEI phosphopeptide (SEQ ID NO 1), and the functional inhibition of Eng, using the siRNA of SEQ ID NO 2, in the context of a ROS-dependent stimulation of cell function. The basal activation of cell proliferation induced by transient induction of ROS production, through phototreatments (PT) with mALA and red light, is significantly enhanced by a combination with activators of Src kinase function (aSrc; YEEI phosphopeptide) and/or an Endoglin function inhibitor (iEng; an siRNA of SEQ ID NO 2 recognizing the *Eng* mRNA), in human primary fibroblasts. The results are measured, 48 hours after the irradiation treatment. The mean +/- SEM of three independent experiments is represented. Statistical significance is referred to Control, except when indicated (*, p<0.1; **, p<0.05 ; *** p<0.001).

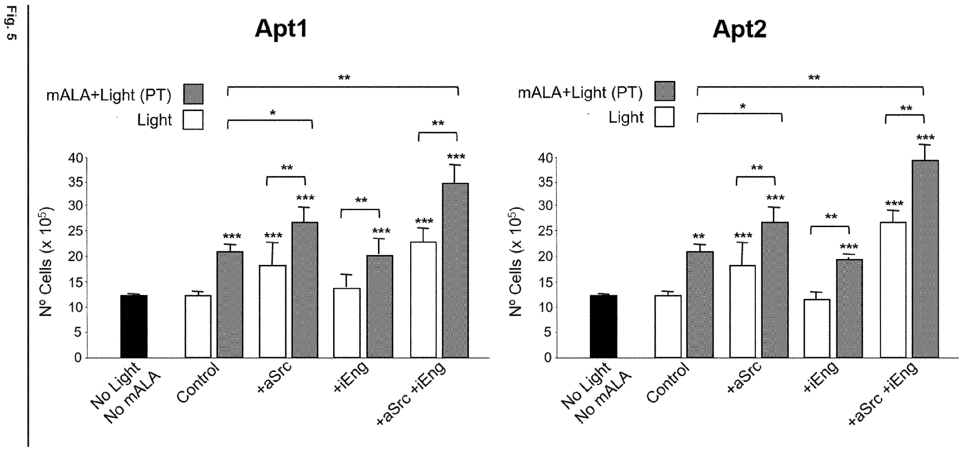

**Figure 5****.** Example showing the biological synergy of the activation of the cSrc kinase, using the YEEI phosphopeptide of SEQ ID NO 1, and the functional inhibition of Eng, the DNA aptamers of SEQ ID NO 3 and 4, in the context of a ROS-dependent stimulation of cell function. The basal activation of cell proliferation induced by transient induction of ROS production, through phototreatments (PT) with mALA and red light, is significantly enhanced by a combination with activators of Src kinase function (aSrc; YEEI phosphopeptide) and/or an Endoglin function inhibitor (iEng; two different aptamers, Apt1 (SEQ ID NO 3) and Apt2 (SEQ ID NO 4), recognizing the tertiary structure of the Eng protein), in human primary fibroblasts. The results are measured, 48 hours after the irradiation treatment. The mean +/- SEM of three independent experiments is represented. Statistical significance is referred to Control, except when indicated (*, p<0.1; **, p<0.05 ; *** p<0.001).

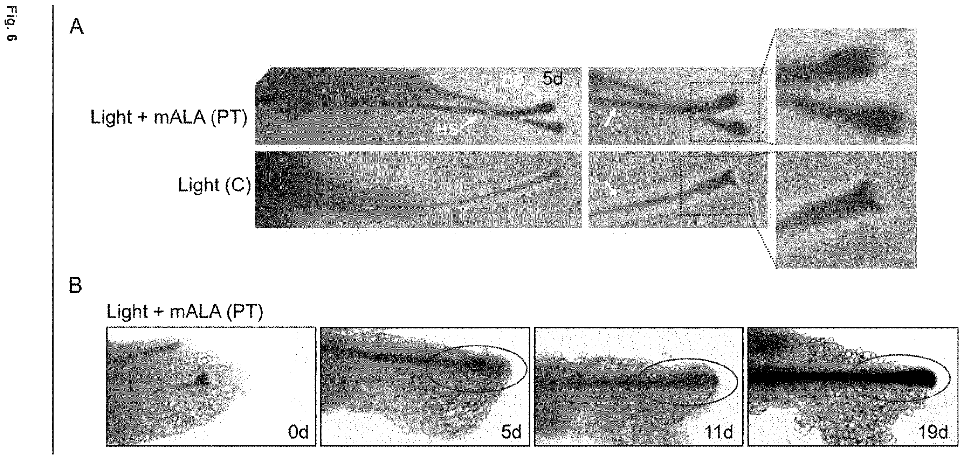

**Figure 6****.** Induction of significant hair bulb/dermal papilla region (DP) thickening in *ex vivo* grown human hair follicles by phototreatments (PT) using mALA and red light. DP thickening is an indicator the activation of the proliferation and differentiation programs of hair follicle stem cell niches. HF activation is consistently evident 5d after PT as compared to control. HS thickening occurs between days 11 and 19. Results are representative of at least 20 different experimental series each including at least 5 different hair follicles.

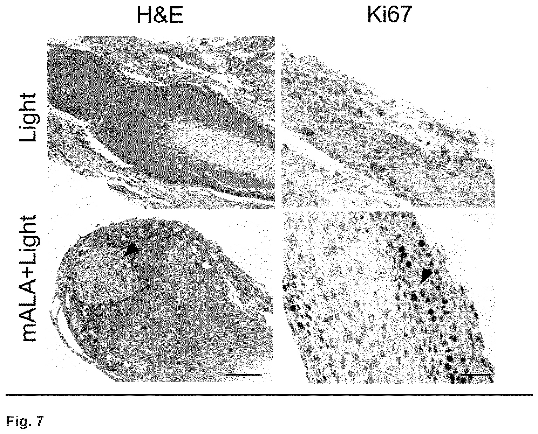

**Figure 7****.** Activation of *ex vivo* grown human hair follicles (HF) by phototreatments (PT) using mALA and red light is associated with an enlargement and significant cell number increase of the dermal papilla (left panels, arrowheads: bars: 100 µm) and with the strong induction of cell proliferation markers (Ki67) in the inner and outer root sheaths of the hair follicle (right panels, arrowhead; bars: 50 µm).

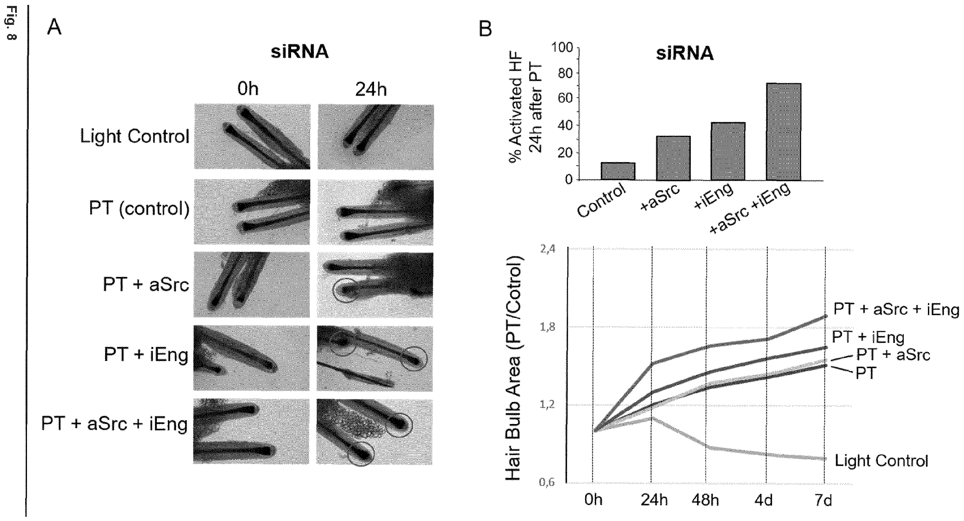

**Figure 8****.** Example showing the biological synergy of the activation of the cSrc kinase, using the YEEI phosphopeptide of SEQ ID NO 1, and the functional inhibition of Eng, using the siRNA of SEQ ID NO 2, in the context of a ROS-dependent stimulation of stem cell/tissue function in a complex mini-organ. A combination of a phototreatment (mALA and red light, PT) with activators of Src kinase function (aSrc; YEEI phosphopeptide) and an Endoglin function inhibitor (iEng; the siRNA recognizing the *Eng* mRNA of SEQ ID NO 2), significantly promotes proliferation and differentiation of the hair bulb area (a marker of hair follicle stem cell activation) just 24 hours after treatments. A) Representative images of hair follicles under different conditions 0 and 24 hours after treatments; red circles indicate activated hair follicles. B) Upper panels: Quantification of the % of activated hair follicles 24 hours after different treatments. 10 hair follicles were analyzed for each treatment. Lower panels: Quantification of the Hair Bulb Area at indicated times after siRNA treatments. Total hair bulb area in high resolution images of whole hair follicles (n=10 in two different experiments) was spotted and quantified through image analysis.

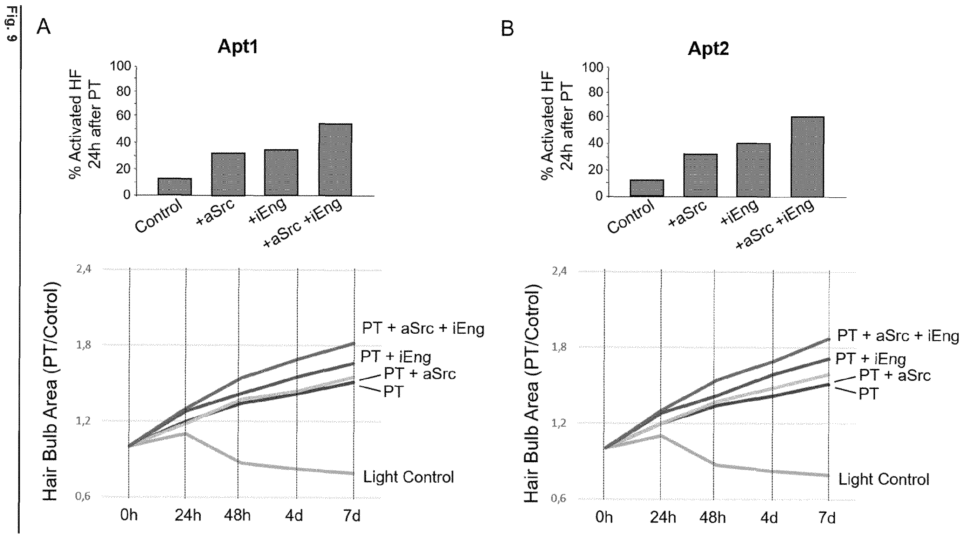

**Figure 9****.** Example showing the biological synergy of the activation of the cSrc kinase, using the YEEI phosphopeptide, and the functional inhibition of Eng, using DNA aptamers, in the context of a ROS-dependent stimulation of stem cell/tissue function in a complex mini-organ. A combination of a phototreatment (mALA and red light, PT) with activators of Src kinase function (aSrc; YEEI phosphopeptide) and an Endoglin function inhibitor (iEng; two different aptamers, Apt1 and Apt2, recognizing the tertiary structure of the Eng protein), significantly promotes proliferation and differentiation of the hair bulb area (a marker of hair follicle stem cell activation) just 24 hours after treatments. Upper panels: Quantification of the % of activated hair follicles 24 hours after Apt1 (A) or Apt2 treatments. 10 hair follicles were analyzed for each treatment. Lower panels: Quantification of the Hair Bulb Area at the indicated times after Apt1 (A) or Apt2 treatments. Total hair bulb area in high resolution images of whole hair follicles (n=10 in two different experiments) was spotted and quantified through image analysis.

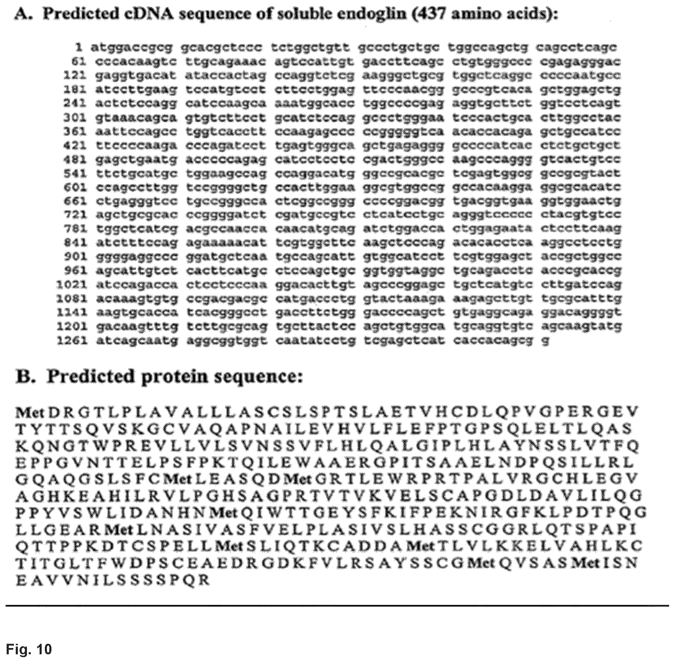

**Figure 10****.** A) Predicted cDNA sequence of soluble endoglin (437 amino acids), B) Predicted protein sequence of soluble endoglin.

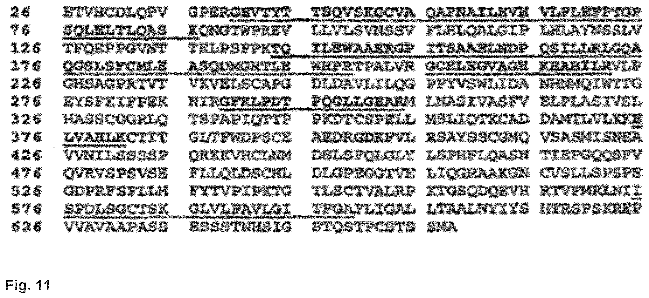

**Figure 11****.** Additional forms of soluble endoglin of the invention that include a protein substantially identical to aminoacids 40(glycine) to 406 (arginine), amino acids 26 (glutamate) to 437 (arginine), amino acids 26 (glutamate) to 587 (leucine) of the human endoglin sequence illustrated in this figure.

### DETAILED DESCRIPTION OF THE INVENTION

### DEFINITIONS

In the context of the present invention "skin homeostasis" is understood as the ongoing renewal of the skin epidermis and its array of appendages. Stem cells in the epidermis have a crucial role in maintaining tissue homeostasis by providing new cells to replace those that are constantly lost during tissue turnover or following injury. Different resident skin stem cell pools contribute to the maintenance and repair of the various epidermal tissues of the skin, including interfollicular epidermis, hair follicles and sebaceous glands. Interestingly, the basic mechanisms and signalling pathways that orchestrate epithelial morphogenesis in the skin are reused during adult life to regulate skin homeostasis.

By "endoglin" or "Eng" also known as CD105, is meant a mammalian growth factor that has endoglin biological activity (see, Fonsatti et al., Oncogene 22:6557-6563, 2003; Fonsatti et al., Curl: Cancer Drug Targets 3:427-432, 2003) and is homologous to the protein defined by any of the following GenBank accession numbers: AAH29080 and NPi031958 (mouse); AAS67893 (rat); NPi000109, P17813, VSPi004233, and CAA80673 (pig); CAA50891 and AAC63386 (human); or those described in US. Pat. No. 6,562,957. Endoglin is a homodimeric cell membrane glycoprotein which is expressed at high levels in proliferating vascular cells and syncytio trophoblasts from placentas. There are two distinct isoforms of endoglin, L and S, which differ in their cytoplasmic tails by 47 amino acids. Both isoforms are included in the term endoglin as used herein. Endoglin is an auxiliary component of the TGF-β receptor system, able to associate with the signaling receptor types I (TGF- β receptor 1;TBRI) and H (TGF- β receptor-2;TβRII) in the presence of ligand and to modulate the cellular responses to TGF- β. Endoglin binds to TGF- β family members and, in the presence of TGF-β, endoglin can associate with TβRI and TβRII, and potentiate the response to the growth factors. Endoglin biological activities include binding to TGF-β family members such as activin-A, BMP-2, BMP-7, TGF- and GF; binding to TGF-β receptors (e.g., TβRI and TβRII); induction of angiogenesis, regulation of cell proliferation, attachment, migration, invasion; and activation of endothelial cells. Assays for endoglin biological activities are known in the art and include ligand binding assays or Scatchard plot analysis; BrdU labeling, cell counting experiments, or quantitative assays for DNA synthesis such as 3H-thymidine incorporation used to measure cell proliferation; and angiogenesis assays such as those described herein or in McCarty et al., Inzl.J.Oncol.21:5-10,2002;Akhtaretal.,Clin.Chem. 49:32 40, 2003; and Yamashita et al., J.Biol.Chem. 269:1995-2001, 1994.

In the context of the present invention, the term functional inhibition of the BMP/Smad co-receptor Endoglin (Eng) has been defined entirely on straightforward operational terms at the gene expression level as the transcriptional repression of two key target genes Id1 and Id2 of Eng signalling in stem cells, the skin and the hair follicle (Calvo-Sanchez MI et al. J Mol Cell Biol 11:39,2019). To achieve a significant inhibition of Eng function in different experimental systems (mouse ES cells, human skin primary cells and human hair follicles grown *ex vivo*)),

By "soluble endoglin polypeptide" or "sEng" is meant any circulating, non-membrane bound form of endoglin which includes at least a part of the extracelular portion of the endoglin protein and is substantially identical (e.g., at least 60%, 70%, 80%, 90%, 95%, 96%, 97%, 98%, 99%, or 100% identical) to the amino acid sequence encoding the extra cellular portion of the endoglin protein (see FIGS.1 and 3B of US20090170767). Soluble endoglin can result from the cleavage of the membrane-bound form of endoglin by a proteolytic enzyme. One potential cleavage site is at amino acid 437 of human endoglin producing a soluble endoglin polypeptide that includes aminoacids 1-437 of the endoglin polypeptide (see, FIGS. 7A and 7B), or a protein that is substantially identical to amino acids 1-437 of the endoglin polypeptide. Additional forms of soluble endoglin of the invention include a protein substantially identical to aminoacids 40(glycine) to 406 (arginine), amino acids 26 (glutamate) to 437 (arginine), amino acids 26 (glutamate) to 587 (leucine) of the human endoglin shown in FIG. 8 of; a protein substantially identical to amino acids 1 to 587 of human endoglin (commercially available from R&D Systems, catalog number 1097-EN); any polypeptide that includes one or more of the peptides identified in bold and underlined in FIG. 8: amino acids 40 (glycine) to 86(lysine); aminoacids 144(threonine) to 199(argi nine); amino acids 206 (glycine) to 222 (arginine); aminoacids 289 (glycine) to 304 (arginine); amino acids 375 (glutamate) to 381 (lysine); and any polypeptide that includes the regions or domains of soluble endoglin that are required for binding to TGF-β (e.g., TGF-β1 and TGF-β3) or TGF-β receptors (e.g., TβRI and TβRII). It should be noted that the numbering of both endoglin and soluble endoglin depends on whether the leader peptide sequence is included.

The numbering of endoglin used herein is shown in FIG. 8, starting at amino acid 26 (Where the absent leader peptide sequence would be amino acids 1-25). Soluble endoglin can also include circulating degradation products or fragments that result from enzymatic cleavage of endoglin and that maintain soluble endoglin biological activity. Preferred soluble endoglin polypeptides have soluble endoglin biological activity such as binding to substrates such as TGF-β family members (e.g., TGF-β1 and TGF-b3) or TGF-b receptors (e.g., TbRI and TBRII) or reversing or inhibiting angiogenesis by at least 10%, 20%, 30%, 40%, 50%, 60%, 70%, 80%, 90%, or more. Examples of assays for measuring these activities are known in the art and described in US. Patent Application Publication Nos. 20060067937 and 20050267021, and PCT Publication No. WO 06/034507, incorporated herein by reference. For example, soluble endoglin biological activity can include the ability to reverse, reduce, or inhibit angiogenesis induced by TGF-b or the ability to reverse activation of Smad 2/3 or Smad 2/3-dependent transcriptional activation. Soluble endoglin polypeptides may be isolated from a variety of sources, such as from mammalian tissue or cells (e.g., placental tissue or cells), or prepared by recombinant or synthetic methods. The term soluble endoglin also encompasses modifications to the polypeptide, fragments, derivatives, analogs, and variants of the endoglin polypeptide.

By "sample" is meant a bodily fluid (e.g., urine, blood, serum, plasma, or cerebrospinal fluid), tissue, or cell.

By "subject" is meant a mammal, including, but not limited to, a human or non-human mammal, such as a bovine, equine, canine, ovine, or feline.

In the context of the present invention, a potentially useful "Src kinase activator" may be the YEEI phosphopeptide; EPQpYEEIPIYL; YEEI peptide. The YEEI peptide contains the following amino acid sequence: Glu-Pro-Gln-Tyr(PO3H2)-Glu-Glu-Ile-Pro-Ile-Tyr-Leu (SEQ ID NO 1).

It is noted with respect to the YEEI peptide, that modified peptides of the YEEI peptide such as phosphopeptides with single amino-acid changes in the four residues C-terminal to the phosphotyrosine (pTyr) in pY324 can also be used in the present invention. In this sense, it is noted that the Glu one residue C-terminal to the phosphotyrosine (at position pY+1) is sensitive to substitution, whereas the Ile at position pY+3 is much less sensitive, accommodating a Glu with only modest loss of binding affinity. Replacement of the Glu and Pro on either side of the Ile had little effect. Truncated phosphopeptides that end at position pY+5 and have only an acetyl group N-terminal to the pTyr bound with only slightly lower affinity than pY324.

By "substantially identical" is meant a nucleic acid or amino acid sequence that, when optimally aligned, for example using the methods described below, share at least 60%, 65%, 70%, 75%, 80%, 85%, 90%, 95%, 96%, 97%, 98%, 99%, or 100% sequence identity with a second nucleic acid or amino acid sequence, e.g., an endoglin or soluble endoglin sequence.

"Substantial identity" may be used to refer to various types and lengths of sequence, such as full length sequence, epitopes or immunogenic peptides, functional domains, coding and/or regulatory sequences, exons, introns, promoters, and genomic sequences. Percent identity between two polypeptides or nucleic acid sequences is determined in various ways that are within the skill in the art, for instance, using publicly available computer software such as Smith Waterman Alignment (Smith and Waterman .1.Mol. Biol. 147:195-7, 1981); "BestFit" (Smith and Waterman, Advances in Applied Mathematics, 482-489, 1981) as incorporated into GeneMatcher PlusTM, SchWarZ and Dayhof "Atlas of Protein Sequence and Structure," Dayhof, M. 0., Ed.,pp353-358,1979; BLASTprogram(BasicLocalAlign ment Search Tool; Altschul et al.,J. Mol. Biol. 215: 403-410, 1990), BLAST-2, BLAST-P, BLAST-N, BLAST-X, WU-BLAST-2, ALIGN, ALIGN-2, CLUSTAL, or Megalign (DNASTAR) software. In addition, those skilled in the art can determine appropriate parameters for measuring alignment, including any algorithms needed to achieve maximal alignment over the length of the sequences being compared. In general, for proteins, the length of comparison sequences will be at least 10, 20, 30, 40, 50, 60, 70, 80, 90, 100, 150, 200, 250, 300, 350, 400, 450, 500, 550, 600, 640 amino acids or more. For nucleic acids, the length of comparison sequences will generally be at least 10, 20, 30, 40, 50, 60, 70, 80, 90, or at least 1500, 1750, 1800 or more nucleotides. It is understood that for the purposes of determining sequence identity when comparing a DNA sequence to an RNA sequence, a thymine nucleotide is equivalent to a uracil nucleotide. Conservative substitutions typically include substitutions within the following groups: glycine, alanine; valine, isoleucine, leucine; asparticacid, glutamicacid, asparagine, glutamine; serine, threonine; lysine, arginine; and phenylalanine, tyrosine.

By "fragment" is meant a portion of a polypeptide or nucleic acid molecule that contains, preferably, at least 10%, 20%, 30%, 40%, 50%, 60%, 70%, 80%, 90%, 95%, or more of the entire length of the reference nucleic acid molecule or polypeptide. A fragment may contain at least 10, 20, 30, 40, 50, 60, 70, 80, 90, or at least 100, 200, 300, 400, 500, 600, 700, 800, 900, 1000, 1250, 1500, 1750, 1800 or more nucleotides or at least 10, 20, 30, 40, 50, 60, 70, 80, 90, 100, 150, 200, 250, 300, 350, 400, 450, 500, 550, 600, 640 amino acids or more. Preferred fragments of soluble endoglin will have soluble endoglin biological activity (e.g., binding to TGF-β or TGF-β receptor) and may include, for example, the TGF-β or TGF-β receptor binding domain.

In the context of the present invention, "Epitope" refers to that portion of an antigen or other macromolecule capable of forming a binding interaction with the variable region binding pocket of an antibody. Such binding interactions can be manifested as an intermolecular contact with one or more amino acid residues of one or more CDRs. Antigen binding can involve, for example, a CDR3 or a CDR3 pair or, in some cases, interactions of up to all six CDRs of the VH and VL chains. An epitope can be a linear peptide sequence (i.e., "continuous") or can be composed of non-contiguous amino acid sequences (i.e., "conformational" or "discontinuous"). An antibody can recognize one or more amino acid sequences; therefore, an epitope can define more than one distinct amino acid sequence. Epitopes recognized by antibodies can be determined by peptide mapping and sequence analysis techniques well known to one of skill in the art. Binding interactions are manifested as intermolecular contacts with one or more amino acid residues of a CDR. Antibodies as the murine antibodies described as Y4-2F1 or SN6j in U.S. Pat. Nos. 5,928,641; 6,200,566; 6,190,660; and 7,097,836. Epitopes recognized by Y4-2F1 and SN6j, have been previously identified.

As used herein, the term "antibody" refers to an immunoglobulin (Ig) whether natural or partly or wholly synthetically produced. The term also covers any polypeptide or protein having a binding domain which is, or is homologous to, an antigen-binding domain. The term further includes "antigen-binding fragments" and other interchangeable terms for similar binding fragments such as described below. Complementarity determining region (CDR) grafted antibodies and other humanized antibodies (including CDR modifications and framework region modifications) are also contemplated by this term.

Other features and advantages of the invention will be apparent from the following description, the drawings, and the claims.

### DESCRIPTION

The authors of the present invention have found that a composition comprising a) a Src kinase activator and b) an Eng function inhibitor are especially useful in a method of treatment which comprises the activation of a population of skin or epidermal stem cells and/or progenitor cells in a subject, preferably in a human subject.

In this sense, and as illustrated in the examples, in a E14 mouse embryonic experimental cell model, the authors of the present invention have demonstrated the ability of the Src kinase activator the YEEI peptide in combination with Eng function inhibitors (iEng) such as, but not limited to, the soluble extracellular domain of the human Eng protein, to regulate the functioning of a pluripotent stem cell system that broadly resembles the stem cell system present in the niches of skin and hair follicles. Such experimental model confirmed that photodynamic treatment with mALA and red light is capable of activating skin or epidermal stem cells proliferation. Interestingly, such activation is **markedly enhanced** by combining said photodynamic procedure with the Src kinase function activator the YEEI peptide and an Endoglin inhibitor (the soluble extracellular domain of the human Eng protein of SEQ ID NO 5) (Figure 3).

In addition, cultures of primary human skin fibroblasts, as illustrated in the examples, indicate that, photodynamic treatment with mALA and red light significantly activates cell proliferation, and such activation is markedly increased by combining the photodynamic procedure with the specific Src kinase function activator the YEEI peptide and Endoglin inhibitors such as, but not limited to, the siRNA of SEQ ID NO 2 or aptamers (Apt1 and Apt2)(Figures 4 and 5).

Furthermore, in *ex vivo* cultures of human hair follicles, the results shown in the examples indicate that the photodynamic treatment with mALA and red light promotes a consistent and strongly significant thickening of the hair bulb/dermal papilla region. This thickening, an indicator of the activation of the stem cell proliferation and differentiation programs driven hair growth, occurs approximately 5 days after treatment (Figure 6) and is consequently associated with the increase of cell numbers in the dermal papilla and the induction of cell proliferation markers in the inner and outer root sheaths (Figure 7). Notably, the combination of the above said photodynamic treatment with the specific Src kinase function activator the YEEI peptide and Endoglin inhibitors such as, but not limited to, the siRNA of SEQ ID NO 2 or aptamers (Apt1 and Apt2), significantly boosts the thickening of the hair bulb/dermal papilla region showing a strong synergistic effect for the induction of hair follicle growth (Figures 8 and 9).

Consequently, the combination of one or more photosensitive agent capable of producing reactive oxygen species (ROS), such as 5-aminolevulinic acid (ALA) or its methylated derivative methyl aminolevulinate (mALA), with a Src kinase function activator and an Endoglin inhibitor:
1. markedly enhances the activation of skin or epidermal stem cell proliferation, significantly activates the proliferation of fibroblasts and potentiates skin homeostasis; and
2. Significantly reduces the activation time and significantly promotes the thickening of hair follicles in the growth phase.

Therefore, a first aspect of the invention refers to a composition comprising a Src kinase activator and an Eng function inhibitor (iEng) or a TGF-β superfamily function inhibitor including activins such as activin-A, or bone morphogenic proteins such as bone morphogenic proteins-2 (BMP-2) and BMP-7, wherein the Src kinase activator activates the cSrc kinase by phosphorylating the Tyr419 residue of the cSrc protein, and wherein the Eng function inhibitor (iEng) is capable of inhibiting the Eng function by reducing the gene expression level of the two key target genes Id1 and Id2 of Eng signalling in skin cells such as skin stem cells, fibroblasts or keratinocytes, in at least 80%, as measure by a gene expression quantification technique such as RT-qPCR, in comparison to said gene expression level of the same the cells not exposed to Eng function inhibitor. It is noted that gene expression quantification of the specific Eng functional targets in the Id1 and Id2 genes is known and can be performed by qRT-PCR as previously described in Fernández-Martos S et al. Int J Mol Sci 20 (7) pii: E1741, 2019; Calvo-Sánchez M et al. Sci Rep 9 (1): 4509, 2019).

It is important to highlight that, in the context of the present invention and as shown in the figures and the examples included in the present specification, the specific functional inhibition of the BMP/Smad co-receptor Endoglin (Eng) has been defined entirely on straightforward operational terms at the gene expression level as the transcriptional repression of two key target genes Id1 and Id2 of Eng signalling in stem cells, the skin and the hair follicle (Calvo-Sanchez MI et al. J Mol Cell Biol 11:39,2019). To achieve a significant inhibition of Eng function in different experimental systems (mouse ES cells, human skin primary cells and human hair follicles grown *ex vivo),* we have used four different approaches:
1. - By using **SEQ ID NO 3.** Aptamer1 (see figures 5 and 9);
2. - By using **SEQ ID NO 4.** Aptamer2 (see figures 5 and 9);
3. - By using **SEQ ID NO 2.** siRNA (see figures 4 and 8); and
4. - By using the overexpression of secreted/soluble form of human S-Endoglin acting as ligand antagonist: cell/tissue lipofection of the pcEXV-EndoS plasmid (Bellon T et al. Eur J Immunol 23:2340, 1993; Letamendia A et al. J Biol Chem 273:33011, 1998) (see figure 3).

The gene expression quantification of the specific Eng functional targets in the Id1 and Id2 genes was performed by qRT-PCR as previously described (Fernández-Martos S et al. Int J Mol Sci 20 (7) pii: E1741, 2019; Calvo-Sánchez M et al. Sci Rep 9 (1): 4509, 2019). **All four approaches have shown essentially the same results in all experimental systems used and, therefore,** in **the context of the present invention, all molecules herein mentioned capable of the transcriptional repression of two key target genes Id1 and Id2 of Eng signalling in skin stem cells, and/or the skin fibroblasts or keratinocytes will be refer from this point on under the term "Eng function inhibitor (iEng)".**

On the other hand, in the context of the present invention and as shown in the figures, the functional activation of the cSrc kinase has been specifically associated with the phosphorilation of the Tyr419 residue (or equivalent) of the cSrc protein (Espada J and Martin-Perez J. Int Rev Cell Mol Biol 331:82, 2017). To achieve full cSRC activation in different experimental systems (mouse ES cells, human skin primary cells and human hair follicles grown *ex vivo),* we have used the YEEI phosphopeptide of SEQ ID NO 1.

Thus, in a particularly preferred embodiment of the first aspect of the present invention or of any of its preferred embodiments, the Src kinase activator is the YEEI phosphopeptide: EPQpYEEIPIYL (SEQ ID NO 1); YEEI peptide or any modifications of this peptide as described in the "Definitions" of the present invention.

In addition, in another preferred embodiment of the first aspect of the invention, the Eng function inhibitor (iEng) is selected from the list consisting of: i) an endoglin binding agent, such as an antibody or antigen-binding fragment thereof or an aptamer that binds to endoglin; ii) a soluble endoglin molecule such as the recombinant glycosylated polypeptide encompassing the soluble extracellular domain of the human Eng protein of SEQ ID NO 5 or a peptide that, when optimally aligned with SEQ ID NO 5, shares at least 60%, 65%, 70%, 75%, 80%, 85%, 90%, 95%, 96%, 97%, 98%, 99% sequence identity with SEQ ID NO 5 and is capable inhibiting the Eng function by reducing the gene expression level of the two key target genes Id1 and Id2 of Eng signalling in skin cells such as skin stem cells, or fibroblasts, in at least 80%, as measure by a gene expression quantification technique such as RT-qPCR, in comparison to said gene expression level of the same the cells not exposed to Eng function inhibitor; or iii) the short interfering RNA or silencing RNA (siRNA) of SEQ ID NO 2 or a siRNA that, when optimally aligned with any of SEQ ID NO 2, shares at least 60%, 65%, 70%, 75%, 80%, 85%, 90%, 95%, 96%, 97%, 98%, 99% sequence identity with SEQ ID NO 2 and is capable inhibiting the Eng function by reducing the gene expression level of the two key target genes Id1 and Id2 of Eng signalling in skin cells such as skin stem cells, or fibroblasts, in at least 80%, as measure by a gene expression quantification technique such as RT-qPCR, in comparison to said gene expression level of the same the cells not exposed to Eng function inhibitor.

In another preferred embodiment of the first aspect of the present invention, the Eng function inhibitor is a soluble endoglin polypeptide. Preferably, said soluble endoglin can result from the cleavage of the membrane-bound form of endoglin by a proteolytic enzyme. One potential cleavage site is at amino acid 437 of human endoglin producing a soluble endoglin polypeptide that includes aminoacids 1-437 of the endoglin polypeptide (see, FIGS. 10A and 10B), or a protein that is substantially identical to amino acids 1-437 of the endoglin polypeptide. Additional forms of soluble endoglin of the invention include a protein substantially identical to aminoacids 40 (glycine) to 406 (arginine), amino acids 26 (glutamate) to 437 (arginine), amino acids 26 (glutamate) to 587 (leucine) of the human endoglin shown in FIG. 11; a protein substantially identical to amino acids 1 to 587 of human endoglin (commercially available from R&D Systems, catalog number 1097-EN); any polypeptide that includes one or more of the peptides identified in bold and underlined in FIG. 11: amino acids 40 (glycine) to 86 (lysine); aminoacids 144 (threonine) to 199 (arginine); amino acids 206 (glycine) to 222 (arginine); amino acids 289 (glycine) to 304 (arginine); amino acids 375 (glutamate) to 381 (lysine); and any polypeptide that includes the regions or domains of soluble endoglin that are required for binding to TGF-β (e.g., TGF-β1 and TGF-β3) or TGF-β receptors (e.g., TβRI and TβRII).

As already stated in the "definitions" above, soluble endoglin polypeptides may be isolated from a variety of sources, such as from mammalian tissue or cells (e.g., placental tissue or cells), or prepared by recombinant or synthetic methods. The term soluble endoglin also encompasses modifications to the polypeptide, fragments, derivatives, analogs, and variants of the endoglin polypeptide.

In another preferred embodiment of the invention, the Eng function inhibitor is a soluble endoglin nucleic acid molecule comprising recombinant glycosylated polypeptide encompassing the soluble extracellular domain of the human Eng protein produced in Sf9 cells (**SEQ ID NO 5**).

In yet another preferred embodiment of the first aspect of the present invention, the Eng function inhibitor is a soluble endoglin nucleic acid molecule comprising a sequence that encodes a polypeptide having a sequence substantially identical to aminoacids 1-437 of the endoglin polypeptide (see FIGS. 10A and 10B), or a protein that is substantially identical to amino acids 1-437 of the endoglin polypeptide.

In another preferred embodiment of the first aspect of the present invention, the Eng function inhibitor is the siRNA consisting of SEQ ID NO 2.

In yet another preferred embodiment of the first aspect of the present invention, the Eng function inhibitor is an endoglin binding agent such as an antibody or an antigen-binding fragment thereof or an aptamer that binds to endoglin, preferably to an endoglin epitope, more preferably to an endoglin epitope recognized by the murine antibodies described as Y4-2F1 or SN6j, wherein said endoglin binding agent is capable of inhibiting the Eng function by reducing the gene expression level of the two key target genes Id1 and Id2 of Eng signalling in skin cells such as skin stem cells, or fibroblasts, in at least 80%, 85%, 90%, 95%, 96%, 97%, 98% or 99%, as measure by a quantitative technique such as RT-qPCR, in comparison to said gene expression level of the same the cells not exposed to Eng function inhibitor. More preferably, the Eng function inhibitor is the aptamer, comprising or consisting of, SEQ ID NO 3 or SEQ ID NO 4 or an aptamer that, when optimally aligned with any of SEQ ID NO 3 or SEQ ID NO 4, shares at least 60%, 65%, 70%, 75%, 80%, 85%, 90%, 95%, 96%, 97%, 98%, 99% sequence identity with any of SEQ ID NO 4 or SEQ ID NO 5 and is capable inhibiting the Eng function by reducing the gene expression level of the two key target genes Id1 and Id2 of Eng signalling in skin cells such as skin stem cells, or fibroblasts, in at least 80%, as measure by a gene expression quantification technique such as RT-qPCR, in comparison to said gene expression level of the same the cells not exposed to Eng function inhibitor.

Exemplary antibodies for use in the compositions and methods described herein are intact immunoglobulin molecules, such as, for example, a humanized antibody or those portions of a humanized Ig molecule that contain the antigen binding site (i.e., paratope) or a single heavy chain and a single light chain, including those portions known in the art as Fab, Fab', F(ab)', F(ab')2, Fd, scFv, a variable heavy domain, a variable light domain, a variable NAR domain, bi-specific scFv, a bi-specific Fab2, a tri-specific Fab3 and a single chain binding polypeptides and others also referred to as antigen-binding fragments. When constructing an immunoglobulin molecule or fragments thereof, variable regions or portions thereof may be fused to, connected to, or otherwise joined to one or more constant regions or portions thereof to produce any of the antibodies or fragments thereof described herein. This may be accomplished in a variety of ways known in the art, including but not limited to, molecular cloning techniques or direct synthesis of the nucleic acids encoding the molecules. In one exemplary embodiment, the application contemplates a single chain binding polypeptide having a heavy chain variable region, and/or a light chain variable region which binds endoglin and has, optionally, an immunoglobulin Fc region. In one exemplary embodiment, the application contemplates a single chain binding polypeptide having a heavy chain variable region, and/or a light chain variable region which binds endoglin and inhibits angiogenesis and has, optionally, an immunoglobulin Fc region. Such a molecule is a single chain variable fragment optionally having effector function or increased half-life through the presence of the immunoglobulin Fc region. Methods of preparing single chain binding polypeptides are known in the art (e.g., U.S. Patent Application No. 2005/0238646).

In yet another preferred embodiment of the first aspect of the present invention or of any of its preferred embodiments, the composition further comprises a protoporphyrin IX metabolic precursor. Preferably, said protoporphyrin IX metabolic precursor is the aminolevulinic acid ALA or the ALA methylated derivative methyl aminolevulinate.

On a different note, it is further indicated that the compositions identified in the first aspect of the invention, as shown in the examples and figures, are especially suitable for use in i) a method of treatment, in ii) an *in vitro* method and/or in iii) a non-therapeutic method of treatment, wherein all of these methods have in common the activation of a population of skin or epidermal stem cells and/or progenitor cells, understood as skin homeostasis and/or regeneration of a population of skin or epidermal stem cells and/or progenitor cells, preferably in a subject, more preferably in a human subject. Preferably, the population of epidermal stem cells and/or progenitor cells is a population of hair follicle stem cells and/or skin epidermal or progenitor cells. More preferably, said in vitro method, method of treatment, or non-therapeutic use is performed subsequently, preferably immediately after or approx. 1 minute after, or simultaneously to the treatment of a population of skin or epidermal stem cells and/or progenitor cells with photosensitive agents capable of producing reactive oxygen species (ROS), such as 5-aminolevulinic acid (ALA) or its methylated derivative methyl aminolevulinate (mALA) and/or a source of light such as a source of red light. Preferably, subsequently, preferably immediately after or approx. 1 minute after, irradiation with a source of light such as a source of red light.

Therefore, a second aspect of the invention refers to an *in vitro* method for the activation of a population of skin or epidermal stem cells and/or progenitor cells, wherein the method comprises applying to a population of skin or epidermal stem cells and/or progenitor cells an effective amount of a Src kinase activator and an Eng function inhibitor (iEng) or a TGF-β superfamily function inhibitor including activin-A, bone morphogenic protein-2 (BMP-2) and BMP-7, formulated in a physiological acceptable medium. Preferably, the Src kinase activator and the Eng function inhibitor (iEng) or the TGF-β superfamily function inhibitor including activin-A, bone morphogenic protein-2 (BMP-2) or BMP-7, are as defined in any of the preferred embodiments described in the first aspect of the invention. More preferably, the Src kinase activator and the Eng function inhibitor (iEng) or the TGF-β superfamily function inhibitor including activin-A, bone morphogenic protein-2 (BMP-2) or BMP-7, are used in combination with a protoporphyrin IX metabolic precursor; wherein preferably, said protoporphyrin IX metabolic precursor is aminolevulinic acid ALA or the ALA methylated derivative methyl aminolevulinate. More preferably, such in vitro method is performed subsequently, preferably immediately after or approx. 1 minute after, or simultaneously to the treatment of said population of skin or epidermal stem cells and/or progenitor cells with photosensitive agents capable of producing reactive oxygen species (ROS), such as 5-aminolevulinic acid (ALA) or its methylated derivative methyl aminolevulinate (mALA) and/or a source of light such as a source of red light. More preferably, subsequently, preferably immediately after or approx. 1 minute after, irradiation with a source of light such as a source of red light.

In a preferred embodiment of the second aspect of the present invention, such methodology provides for derma or derma equivalents such as skin (artificial skin) with or without hairs, and/or isolated hairs and/or hair follicles, preferably hair follicle stem cells. Therefore, the present invention provides human skin equivalents and compositions and methods for making human skin equivalents. In this sense, successful treatment of chronic skin wounds (e.g., venous ulcers, diabetic ulcers, pressure ulcers) is a serious problem. The healing of such a wound often times takes well over a year of treatment. Treatment options currently include dressings and debridement (use of chemicals or surgery to clear away necrotic tissue), and/or antibiotics in the case of infection. These treatment options take extended periods of time and high amounts of patient compliance. As such, a therapy that can increase a practioner's success in healing chronic wounds and accelerate the rate of wound healing would meet an unmet need in the field. Consequently, in some embodiments, the present invention contemplates treatment of skin wounds with the human skin equivalents obtained or obtainable by the method of the second aspect of the invention.

Furthermore, a third aspect of the invention refers to a pharmaceutical composition comprising an effective amount of a Src kinase activator and an Eng function inhibitor (iEng) or a TGF-β superfamily function inhibitor including activin-A, bone morphogenic protein-2 (BMP-2) and BMP-7, formulated in a pharmaceutically acceptable medium. Preferably, the Src kinase activator and the Eng function inhibitor (iEng) or the TGF-β superfamily function inhibitor including activin-A, bone morphogenic protein-2 (BMP-2) or BMP-7, are as defined in any of the embodiments described in the first aspect of the invention. More preferably, the Src kinase activator and the Eng function inhibitor (iEng) or the TGF-β superfamily function inhibitor including activin-A, bone morphogenic protein-2 (BMP-2) or BMP-7, are used in combination with a protoporphyrin IX metabolic precursor; wherein preferably, said protoporphyrin IX metabolic precursor is aminolevulinic acid ALA or the ALA methylated derivative methyl aminolevulinate.

A fourth aspect of the invention refers to the composition as defined in the first aspect of the invention or the pharmaceutical composition as defined in the third aspect of the invention for use in a method of treatment of a hair loss disorder in a subject in need thereof. Preferably, said composition is for use in stimulating hair growth in a subject suffering of a hair-loss disorder. Preferably, the hair loss is due to androgenetic alopecia (AGA). More preferably, the AGA is male pattern baldness. Still more preferably, the AGA is female pattern baldness. Still more preferably, the hair loss is the result of a skin injury. Still more preferably, the hair loss is in the scalp or eyebrow of said subject. Still more preferably, the hair loss is in scarred skin tissue of said subject. Still more preferably, the scarring alopecia is hair implant scarring alopecia. Still more preferably, the scarring alopecia is acquired by radiation. Still more preferably, the scarring alopecia is frontal fibrosing scarring alopecia. Still more preferably, the hair loss is the result of a non-scarring alopecia. Still more preferably, the non-scarring alopecia is autoimmune non-scarring (Areata) alopecia. Still more preferably, the non-scarring alopecia is non-scarring follicular miniaturization alopecia. Still more preferably, the non-scarring alopecia is Effluviums. More preferably, such therapeutic method is performed subsequently (after) or simultaneously to the administration in the treatment site of said subject with photosensitive agents capable of producing reactive oxygen species (ROS), such as 5-aminolevulinic acid (ALA) or its methylated derivative methyl aminolevulinate (mALA) and/or a source of light such as a source of red light. In particular, subsequently, preferably immediately after or approx. 1 minute after, irradiation with a source of light such as a source of red light.

A fifth aspect of the invention refers to the composition as defined in the first aspect of the invention or the pharmaceutical composition as defined in the third aspect of the present invention, for use in a method of treatment of skin regeneration, preferably wherein said method of treatment is for use in wound and/or burn healing in a subject in need thereof, preferably in a human subject. More preferably, such therapeutic method is performed subsequently or simultaneously to the administration in the treatment site of said subject with photosensitive agents capable of producing reactive oxygen species (ROS), such as 5-aminolevulinic acid (ALA) or its methylated derivative methyl aminolevulinate (mALA) and/or a source of light such as a source of red light. In particular, subsequently, preferably immediately after or approx. 1 minute after, irradiation with a source of light such as a source of red light.

In a preferred embodiment of the fifth aspect of the invention, the said composition or pharmaceutical composition is for use in repairing or ameliorating the adverse effects of the environment, daily stress, sun exposure, or pre-mature aging on human skin, comprising applying to the skin the aforesaid composition. The present invention thus provides methods and compositions for repairing adverse effects of the environment, daily stress, sun exposure, or pre-mature aging on human skin. The term "repairing the adverse effects ... on human skin" is used herein to designate arresting, reversing, ameliorating, diminishing, and/or reducing defects, imperfections, or aesthetically unpleasant conditions of the skin, which include, but are not limited to: age spots, sunburn, sun spots, lines, fine lines, wrinkles, crow's feet, spider veins, stretch marks, dark eye circles, hyperpigmentation, hypopigmentation, discoloration, uneven skin tone, dullness, freckles, skin breakout, blemishes, skin fragility, dryness, patchiness, tactile roughness, chapping, sagginess, thinning, enlarged pores, cellulite formation, acne formation, rosacea, psoriasis, and eczema.

In a preferred embodiment of the fourth or fifth aspect of the present invention or of any of its preferred embodiments, said composition is formulated in a pharmaceutically acceptable medium suitable for topical application, and said composition is preferably administered topically in the treatment site of the subject in need thereof.

A sixth aspect of the invention refers to a cosmetic or pharmaceutical composition comprising a Src kinase activator and an Eng function inhibitor (iEng) or a TGF-β superfamily function inhibitor including activin-A, bone morphogenic protein-2 (BMP-2) and BMP-7. Preferably, the Src kinase activator and the Eng function inhibitor (iEng) or the TGF-β superfamily function inhibitor including activin-A, bone morphogenic protein-2 (BMP-2) or BMP-7, are as defined in any of the embodiments described in the first aspect of the invention. More preferably, further to the Src kinase activator and the Eng function inhibitor (iEng) or the TGF-β superfamily function inhibitor including activin-A, bone morphogenic protein-2 (BMP-2) or BMP-7, the said composition may comprise a protoporphyrin IX metabolic precursor; wherein preferably, said protoporphyrin IX metabolic precursor is aminolevulinic acid ALA or the ALA methylated derivative methyl aminolevulinate.

A seventh aspect of the invention refers to the non-therapeutic use of a cosmetic composition as defined in the sixth aspect of the invention, in the physiological activation of a population of skin or epidermal stem cells in a subject, preferably in a human subject. Preferably, further to the Src kinase activator and the Eng function inhibitor (iEng) or the TGF-β superfamily function inhibitor including activin-A, bone morphogenic protein-2 (BMP-2) or BMP-7, the said composition may comprise a protoporphyrin IX metabolic precursor; wherein preferably, said protoporphyrin IX metabolic precursor is aminolevulinic acid ALA or the ALA methylated derivative methyl aminolevulinate. More preferably, such non-therapeutic method is performed subsequently or simultaneously to the treatment of said population of skin or epidermal stem cells with photosensitive agents capable of producing reactive oxygen species (ROS), such as 5-aminolevulinic acid (ALA) or its methylated derivative methyl aminolevulinate (mALA) and/or a source of light such as a source of red light. In particular subsequently, preferably immediately after or approx. 1 minute after, irradiation with a source of light such as a source of red light.

In a preferred embodiment of the seventh aspect of the invention, said cosmetic composition is use in the physiological activation, understood as skin homeostasis and/or regeneration, of a population of skin or epidermal stem cells and/or progenitor cells in a subject, preferably in a human subject.

In another preferred embodiment of the seventh aspect of the invention, the said cosmetic composition is for use in repairing or ameliorating the adverse effects of the environment, daily stress, sun exposure, or pre-mature aging on human skin, comprising applying to the skin the aforesaid composition. The present invention thus provides methods and compositions for repairing adverse effects of the environment, daily stress, sun exposure, or pre-mature aging on human skin. The term "repairing the adverse effects ... on human skin" is used herein to designate arresting, reversing, ameliorating, diminishing, and/or reducing defects, imperfections, or aesthetically unpleasant conditions of the skin, which include, but are not limited to: age spots, sunburn, sun spots, lines, fine lines, wrinkles, crow's feet, spider veins, stretch marks, dark eye circles, hyperpigmentation, hypopigmentation, discoloration, uneven skin tone, dullness, freckles, skin breakout, blemishes, skin fragility, dryness, patchiness, tactile roughness, chapping, sagginess, thinning, enlarged pores, cellulite formation, acne formation, rosacea, psoriasis, and eczema.

In another preferred embodiment of the seventh aspect of the invention, the population of epidermal stem cells and/or progenitor cells is a population of hair follicle stem cells and/or progenitor cells.

In another preferred embodiment of the seventh aspect of the invention, said activation is for use in hair follicle regeneration. Preferably, said composition is use for stimulating hair growth in a subject in need thereof, preferably in a subject with a hair loss disorder. Preferably, the hair loss is due to androgenetic alopecia (AGA). More preferably, the AGA is male pattern baldness. Still more preferably, the AGA is female pattern baldness. Still more preferably, the hair loss is the result of a skin injury. Still more preferably, the hair loss is in the scalp or eyebrow of said subject.

Still more preferably, the hair loss is in scarred skin tissue of said subject. Still more preferably, the scarring alopecia is hair implant scarring alopecia. Still more preferably, the scarring alopecia is acquired by radiation. Still more preferably, the scarring alopecia is frontal fibrosing scarring alopecia. Still more preferably, the hair loss is the result of a non-scarring alopecia. Still more preferably, the non-scarring alopecia is autoimmune non-scarring (Areata) alopecia. Still more preferably, the non-scarring alopecia is non-scarring follicular miniaturization alopecia. Still more preferably, the non-scarring alopecia is Effluviums.

In another preferred embodiment of the seventh aspect of the invention, said composition is formulated in a physiologically acceptable medium, wherein preferably said physiologically acceptable medium is suitable for topical application and the composition is preferably administered topically.

In another preferred embodiment of the seventh aspect of the invention, the population of skin or epidermal stem cells is a human stem cell population.

Specifically, any of the cosmetic or pharmaceutical compositions described in the invention may comprise further active ingredients for treating skin with this composition. It is believed that such active ingredients, in a preferably topical composition, act in synergy to boost or enhance the natural repair responses in the skin cells and therefore improve the effectiveness of cellular repair mechanism against adverse effects of the environment, daily stress, sun exposure, or pre-mature aging on human skin. It is also believed that when an individual is resting, the skin of such an individual is more receptive to active ingredients that will help restore and revitalize its appearance, and the natural repair responses in the skin cells can be most effectively boosted or enhanced. Correspondingly, it is desirable to apply the topical composition of the present invention to the skin prior to a period of bodily rest, which can be either a nightly sleep (e.g., from about 3 to about 10 hours) or a nap (e.g., from about 15 minutes to about 4 hours).

The following examples are merely for illustration purposes.

### Examples

### Materials and methods

### Mouse E14 embryonic stem cells culture procedures.

The feeder-independent E14 mouse embryonic stem cells (ES-E14TG2a), human primary keratinocytes, human primary fibroblasts, and human hair follicles cultured *ex vivo*, were used in this procedure.

E14 cell line is a derivative of mice embryonic stem cells (ES) obtained from the Inner Cell Mass of 129/Ola mouse strain blastocyst. E14 cells were grown on 0,1% gelatin-coated flasks in GMEM supplemented with 10% FCS tested for stem cells, 0.1 mM mixture of Non Essential Amino acids (NEAA, composed by Glycine, L-Alanine, L-Asparagine, L-Aspartic Acid, L-Glutamic acid, L-Proline and L-Serine), 2 mM L-Glutamine, 1 mM sodium pyruvate, 0.05 mM β-mercaptoethanol, Penicilin/Streptomicin (100 U/ml / 100 µg/ml) and 2.5 µg/ml amphotericin B (all from Gibco) and 1,000 U/ml LIF (Millipore), and splitted 1:8 to 1:10 every 2-3 days using Trypsin-EDTA (Invitrogen) (Smith, 1991; Cambray et al, 2012). Cells were maintained at 37°C in a 5% CO₂ humidified atmosphere. The medium was changed on alternative days.

### Human skin primary fibroblast culture procedures.

Primary cultures of human fibroblasts were obtained from normal human skin. Collection of skin biopsy punches, 4mm diameter, from volunteer donors was routinely performed in the Dermatology Service of the Ramon y Cajal University Hospital, Madrid, Spain, following the regulations of the hospital ethics committee for human research, eligible patients providing written informed consent.

Skin samples were transferred to the laboratory and maintained at 4 °C in DMEM medium (Gibco) with 10x Penicilin/Streptomycin and 10x Aphotericin B until processed, not exceeding 24h. Then, the subcutaneous layer was removed using scalpels, and skin biopsies were cut in small pieces of 1-4 mm². In order to obtain primary fibroblasts, skin pieces were incubated in 1mg/ml of collagenase A enzyme (Roche) solution for 4 hours at 37 °C, in mild agitation in order to help cell dissociation.

The supernatant containing the fibroblasts was vigorously pipetted and diluted in DMEM medium supplemented with 10% FCS, 2 mM L-Glutamine, Penicilin/Streptomicin (100 U/ml / 100 µg/ml) and 2.5 µg/ml amphotericin B and centrifuged for five minutes at 1500 RPM. The pellet was resuspended in complete DMEM medium and the fibroblasts were seeded in a maximum density of 1 million cells per ml using a 24 well plate and routinely grown at 37°C in a humidified atmosphere containing CO₂ 5%. 24 hours later, medium was removed to eliminate all detached cells. The cells were passaged when they reached 70-80% confluency, in order to prevent differentiation. The primary culture reached appropriate confluence after 8 to 10 days, and the derivative subcultures were splitted 1:4 once a week. After 10 to 20 passages, cells undergo senescence.

### Human hair follicle ex vivo culture procedures.

Human hair follicles were obtained from human scalp samples taken from the occipital skin of volunteer donors during a hair transplant procedure. Eligible patients provided written informed consent, and the Ethical committee of the Ramon y Cajal Hospital approved this procedure.

Follicular units (FUs) were dissected using scalpels under a magnifier, and the ones in resting phase (telogen) were selected. Those FUs were transferred to the laboratory and maintained at 4 °C until processed in Williams' medium E (Sigma) supplemented with 10x Penicilin/Streptomycin, 10x Aphotericin B, and 2 mM L-Glutamin (all from Gibco), 5 µg/ml Insulin, 5 µg/ml transferring, 20 pM T3 hormone, 0.083 µg/ml cholera toxin and 0.4 µg/ml hydrocortisone. FUs can be stored in complete Williams' medium at 4 °C for approximately 5 days without significant loss of the mini organ function. FU cultures were maintained at 37°C in a 5% CO₂ humidified atmosphere.

### Photodynamic treatments in combination with Src kinase activators and Eng function inhibitors.

Endogenous protoporphyrin IX (PpIX)-dependent photodynamic treatments (PT) were routinely performed on exponentially growing cells at 60% of confluence, as well as in ex vivo cultured hair follicles (HF) (Carrasco E et al. Methods 15:109, 2016; Calvo-Sanchez MI et al. Sci. Rep.9:4509, 2019). To that end, cells and HFs were incubated for 2-4 hours, normally 2 hours, with 0.1 mM of a PpIX metabolic precursor (typically, aminolevulinic acid ALA, or the ALA methylated derivative methyl aminolevulinate mALA) in the corresponding medium in the absence of serum or growth factors. Immediately, approximately 1 minute, after the incubation step the samples were further irradiated 2-15 min, normally between 8 and 10 minutes, with a red-light emitting diode source with an emission peak at 634 nm bandwidth, with a total light dose of 2-10 J/cm², normally 4 J/cm² (Carrasco E et al. Methods 15:109, 2016; Calvo-Sanchez MI et al. Sci. Rep.9:4509, 2019). Immediately after irradiation, approx. 1 minute after, samples were washed in PBS, fresh medium was added, and were further routinely grown at 37°C in a humidified atmosphere containing CO₂ 5%. When required, activators of cSrc kinase or inhibitors of Eng function (see below) were added, alone or in combination, in the fresh medium used immediately after irradiation, and samples were subsequently incubated for the indicated times. Light control samples, with no PpIX precursor added, were included in each experimental condition. Additionally, total controls in strict dark conditions (no PpIX precursor, no light) were evaluated for each biological model, although this control was not routinely used in experimental series since ambient light is an unavoidable contaminant, and the light control is the most adequate in this experimental design.

### Activation of the cSrc kinase.

Here the functional activation of the cSrc kinase has been specifically associated with the phosphorilation of the Tyr419 residue (or equivalent) of the cSrc protein (Espada J and Martin-Perez J. Int Rev Cell Mol Biol 331:82, 2017). To achieve full cSRC activation (aSrc) in different experimental systems (mouse ES cells, human skin primary cells and human hair follicles grown *ex vivo*), we have used treatments with the YEEI phosphopeptide (QPGpYQQIYL (SEQ ID NO 1)). For each biological model and experimental series, the YEEI phosphopeptide was added to the corresponding growth media at a final concentration of 100 nM. In the case of experimental series including PpIX-dependent activation of a transient ROS production, the YEEI phosphopeptide was added to the growth medium after phototreatments (see above), alone or in combination with other compounds, and the samples were subsequently incubated for the indicated times. The biological activity of each YEEI phosphopeptide batch was evaluated by immunoblot detection of cSrc-Tyr419 levels in protein extracts obtained from treated cell cultures (see Figure 1)

### Inhibition of Eng function.

Here the specific functional inhibition of the BMP/Smad co-receptor Endoglin (Eng) has been defined entirely on straightforward operational terms at the gene expression level as the transcriptional repression of two key target genes Id1 and Id2 of Eng signalling in stem cells, the skin and the hair follicle (Calvo-Sanchez MI et al. J Mol Cell Biol 11:39,2019). To achieve a significant inhibition of Eng function in different experimental systems (mouse ES cells, human skin primary cells and human hair follicles grown *ex vivo*), three different approaches were used:
- A siRNA targeting the mRNA of human Eng with the following linear sequence (SEQ ID NO 2):

5´CATGCAGATCTGGACCACTGGAGAATACTCCTTCAAGATCTTTCCAGAGAAAAAC
ATTCGTGGCTTCAAGCTCCCAGACACACCTCAAGGCCTCCTGGGGAGGCCCGGATGCTC
AATGCCAGCATTGTGGCATCCTTCGTGGAGCTACCGCTGGCCAGCATTGTCTCACTTCATG
CCTCCAGCTGCGGTGGTAGGCTGCAGACCTCACCCGCACCGATCCAGACCACTCCTCCCA
AGGACACTTGTAGCCCGGAGCTGCTCATGTCCTTGATCCAGACAAAGTGTGCCGACGACG
CCATGACCCTGGTACTAAAGAAAGAGCTTGTTGCGCATTTGAAGTGCACCATCACGGGCCT
GACCTTCTGGGACCCCAGCTGTGAGGCAGAGGACAGGGGTGACAAGTTTGTCTTGCGCA
GTGCTTACT3´

The siRNA was synthesized by Sigma-Aldrich at 250 nM scale, purified by HPLC and supplied lyophilized. For each biological model and experimental series, the siRNA was added to the corresponding growth media at a final concentration of 50 nM. In the case of experimental series including PpIX-dependent activation of a transient ROS production, the siRNA was added to the growth medium after phototreatments (see above), alone or in combination with other compounds, and the samples were subsequently incubated for the indicated times.
- DNA aptamers recognizing the tertiary structure of the Eng protein with the following linear sequences:
   - Aptamer1 (SEQ ID NO 3): 
5´GCGGATGAAGACTGGTGTGGGTGGGTGGGTGGGAAGGCGGCAGGCTTTGGTTC
GCTGGGCCCTAAATACGAGCAAC3´
   - Aptamer2 (SEQ ID NO 4):

5'CCCCCGATGCTTTCGCTTTTCCTTTCGCTTTTGTTCGCTTCGTCCCTGCTTCCTTT
CTTG3'

The DNA aptamers were selected by SELEX (Systematic Evolution of Ligands by Exponential Enrichment) and scale-up by IBA Lifesciences at 200 nM scale and purchased lyophilized. For each biological model and experimental series, each aptamer was added to the corresponding growth media at a final concentration of 200 nM. In the case of experimental series including PpIX-dependent activation of a transient ROS production, the aptamers were added to the growth medium after phototreatments (see above), alone or in combination with other compounds, and the samples were subsequently incubated for the indicated times.
- A recombinant glycosylated polypeptide encompassing the soluble extracellular domain of the human Eng protein (S-Endoglin) acting as ligand antagonist of the full Eng protein with the following sequence (SEQ ID NO 5):

ETVHCDLQPV GPERGEVTYT TSQVSKGCVA QAPNAILEVH VLFLEFPTGP
SQLELTLQAS KQNGTWPREV LLVLSVNSSV FLHLQALGIP LHLAYNSSLV TFQEPPGVNT
TELPSFPKTQ ILEWAAERGP ITSAAELNDP QSILLRLGQA QGSLSFCMLE ASQDMGRTLE
WRPRTPALVR GCHLEGVAGH KEAHILRVLP GHSAGPRTVT VKVELSCAPG DLDAVLILQG
PPYVSWLIDA NHNMQIWTTG EYSFKIFPEK NIRGFKLPDT PQGLLGEARM LNASIVASFV
ELPLASIVSL HASSCGGRLQ TSPAPIQTTP PKDTCSPELL MSLIQTKCAD DAMTLVLKKE
LVAHLKCTIT GLTFWDPSCE AEDRGDKFVL RSAYSSCGMQ VSASMISNEA VVNILSSSSP
QRKKVHCLNM DSLSFQLGLY LSPHFLQASN TIEPGQQSFV QVRVSPSVSE FLLQLDSCHL
DLGPEGGTVE LIQGRAAKGN CVSLLSPSPE GDPRFSFLLH FYTVPIPKTG TLSCTVALRP
KTGSQDQEVH RTVFMRLNII SPDLSGCTSH HHHHH.

The recombinant peptide was synthesized by BD Bioscience at 2 µg scale, purified by HPLC and supplied lyophilized. For each biological model and experimental series, the recombinant protein was added to the corresponding growth media at 0.2-0.4 mg/ml. In the case of experimental series including PpIX-dependent activation of a transient ROS production, the recombinant peptide was added to the growth medium after phototreatments (see above), alone or in combination with other compounds, and the samples were subsequently incubated for the indicated times.

Id1 and Id2 mouse and human gene expression was quantified by qRT-PCR as previously described (see Figure 2; Calvo-Sanchez MI et al. J Mol Cell Biol 11:39,2019).

### Cell counting and quantification of the hair bulb/dermal papilla region area.

For the quantification of the number of cells in cell cultures after different treatments, a Bio-Rad TC20™ automated cell counter was used following the instructions of the manufacturer. For the evaluation of significant morphological changes *in vivo* in whole hair follicles, high resolution images of FUs growing in 24-well plates were acquired at time 0 after PT, and every 24 hours onwards for 16 days, depending on experimental settings. Image acquisition was performed using a Nikon Eclipse Ci LED-fluorescence microscope using a 2x objective and a 0.55x Reduction Lens adapter coupled to a Jenoptik PROGRES GRYPHAX® SUBRA Super HD camera and suited Version 1.1.8.153 image software pack. We have defined the hair bulb area as the hair follicle territory encompassing the hair bulb and dermal papilla as well as the most intensively pigmented area of the suprabulbar region, including associated inner and outer root sheets (see Fig. 1). This area is characterized by high cell proliferation and melanogenesis during the anagen phase of the hair follicle cycle. Total hair bulb area in high resolution images of whole hair follicles was spotted and quantified using suited free FIJI software packs (https://fiji.sc/). The fold changes with respect to control samples of means +/- SD of hair bulb measurements in n ≥ 10 samples for each experimental condition was represented and T-test was used for statistical analysis.

### Immunological procedures

Primary antibodies used include rabbit monoclonal antibodies against human/mouse cSrc and Phospho Tyr419-Src (Cel Signalling) and mouse monoclonal against human/mouse Ki67 and Histone H3.

For immunolocalization of proteins in histological sections, samples were fixed in pH 7.0 buffered 3.7% formaldehyde and processed for histology. Histological sections were stained with the indicated antibodies after permeabilization in 0.1% Triton X-100. The immunofluorescent signal was revealed using HRP-coupled secondary antibodies and TSA Plus Cyanine 3 (Perkin Elmer) for signal amplification, following the manufacturer's instructions. Histological sections were also stained with standard haematoxylin-eosin for routine evaluation of tissue morphology. Confocal images were obtained in Leica TCS SP2 and SP5 AOBS spectral confocal microscope and processed using the FIJI software (Image J 1.49).

For immunoblotting, cell pellets or hair follicle samples were homogenized in RIPA (25 mM Tris-HCI pH 7.6, 150 mM NaCl, 1% NP-40, 0.1% SDS) or SDS buffer (50 mM Tris-HCI pH 6.8, 2% SDS, 10% glycerol, 1% β-mercaptoethanol, 12.5 mM EDTA) containing protease and phosphatase inhibitors (2 µg/ml aprotinin, 2 mM PMSF, 2 µg/ml leupeptin and 2 mM sodium orthovanadate, 2 mM β-glycerophosphate, 5 mM NaF, all from Sigma-Aldrich). Equivalent amounts of protein for each sample were loaded in Laemmli buffer. Proteins were resolved in a 7.5% SDS-PAGE system, and were transferred to a PVDF membrane, which was blocked and stained with the indicated primary and secondary HRP conjugated antibodies. Finally, HRP activity was detected using the ECL-chemiluminescent kit (Amersham) in accordance with the manufacturer's instructions.

### Gene expression procedures

For RNA extraction from cell cultures, RNeasy mini kit and RNase-Free DNase Set (both form Qiagen) were used. For reverse transcription, MLV enzyme (Promega) was used, loading the same amount of RNA. qRT-PCR assays, or semiquantitative PCR, were performed for gene expression analysis, using Power SYBR Green (Applied Biosystems), or REDExtract-N-Amp™ PCR ReadyMix™ (Sigma Aldrich), respectively, following manufacturer's instructions. Specific primers recognizing human and mouse Id1 and Id2 mRNAs were designed among different exons, thereby avoiding residual genomic DNA amplification, for semiquantitative or quantitative transcript detection.

### Statistical analysis

The numerical treatment of results was done with the SPSS 15.0 software package (SPSS Inc., IBM). Both t-Student and Analysis of Variance (ANOVA) tests were employed to evaluate statistical significance of results.

### RESULTS

In the different experimental models used, the activation of cell proliferation markers and the consequent increase in the number of cells are used as biological indicators of the effectiveness of each specific treatment. Moreover, when in the context of the skin and the hair follicle, the activation of the proliferation of these cell types would be directly associated to the skin homeostasis and the regeneration of tissue as well as to the entry of hair in the growth phase. It is noted that skin epidermis and its array of appendages undergo ongoing renewal by a process called homeostasis. Stem cells in the epidermis have a crucial role in maintaining tissue homeostasis by providing new cells to replace those that are constantly lost during tissue turnover or following injury. Different resident skin stem cell pools contribute to the maintenance and repair of the various epidermal tissues of the skin, including interfollicular epidermis, hair follicles and sebaceous glands. Interestingly, the basic mechanisms and signalling pathways that orchestrate epithelial morphogenesis in the skin are reused during adult life to regulate skin homeostasis.

### - E14 mouse embryonic experimental cell model

We used this experimental model as an indicator of the ability of Src kinase activators (of SEQ ID NO 1) in combination with Eng function inhibitors (iEng) (of SEQ ID NO 5), to regulate the functioning of a pluripotent stem cell system that broadly resembles the stem cell system present in the niches of skin and hair follicles.

The experiments illustrated in the first section of the examples by using the E14 mouse embryonic experimental cell model, confirms that photodynamic treatment with mALA and red light is capable of activating proliferation and increase the number of cells with respect to the control. Interestingly, such activation is **markedly enhanced** by combining said photodynamic procedure with a Src kinase function activator (the YEEI peptide of SEQ ID NO 1) and an Endoglin inhibitor (the soluble extracellular domain of the human Eng protein of SEQ ID NO 5) (Figure 3). It is noted that said activation is associated with the Tyr419 phosphorylation of the Src kinase, as an indicator of functional enzymatic activation (Figure 1) and with the inhibition of the Eng co-receptor function, measured as the impairment to transmit the specific molecular signal that ultimately results in the transcriptional activation of two target genes, Id1 and Id2 (Figure 2).

### - Cultures of primary human skin fibroblasts.

We, again, used these primary cell model as a biological indicator of the ability of Src kinase activators in combination with Eng function inhibitors (iEng) or TGF-β superfamily function inhibitors, to regulate the functioning of this major cellular populations of the skin, in particular, as enhancers of skin homeostasis and of the regenerative capacity of the skin.

In this sense, the results obtained thus far indicate that, photodynamic treatment with mALA and red light activates the proliferation of human primary skin fibroblasts. This activation is markedly increased by combining the photodynamic procedure with a Src kinase function activator (the YEEI peptide of SEQ ID NO 1) and different Endoglin inhibitors (Figures 4 (the siRNA targeting the mRNA of human Eng of SEQ ID NO 2) and 5 (two different aptamers, Apt1 and Apt2 of, respectively, SEQ ID NO 3 and SEQ ID NO 4). It is noted, that such activation is associated with the Tyr419 phosphorylation of the Src kinase (Figure 1) and the downregulation of the Id1 and Id2 gene expression (Figure 2).

### - Ex vivo cultures of human hair follicles

We used this experimental model as a direct indicator of the ability of Src kinase activators in combination with Eng function inhibitors (iEng), to regulate the activation of the proliferation and differentiation programs in hair follicle stem cell niches that ultimately lead to the growth and differentiation of this cycling miniorgan.

The results shown herein indicate that the photodynamic treatment with mALA and red light activates consistently tissue stem cell niches resulting in a prolonged thickening of the hair follicle hair bulb/dermal papilla region (Figure 6). This activation occurs approximately 5 days after treatment (Figure 6 and 7) and is associated with a significant in cell numbers and induction of proliferation markers in the hair bulb region and in the inner and outer root sheaths (Figure 7).

Notably, the combination of the above said photodynamic treatment with a Src kinase function activator with different Endoglin inhibitors significantly promotes the delivery of the hair follicles in the growth phase 24 hours after treatment (Figures 8 (the YEEI peptide and the siRNA targeting the mRNA of human Eng of SEQ ID NO 2) and 9 (the YEEI peptide and the two different aptamers, Apt1 and Apt2 of, respectively, SEQ ID NO 3 and SEQ ID NO 4). Therefore, the combination of the above said photodynamic treatment with a Src kinase function activator and an Endoglin inhibitor, significantly reduces the activation time and significantly promotes the activation of hair follicle proliferation and differentiation.

## Claims

1. A composition comprising the YEEI phosphopeptide consisting of SEQ ID NO 1 and an Eng function inhibitor (iEng) capable of inhibiting the Eng function by reducing the gene expression level of the two key target genes Id1 and Id2 of Eng signalling in skin cells in at least 80%, as measure by a gene expression quantification technique such as RT-qPCR, in comparison to said gene expression levels of the same cells not exposed to the Eng function inhibitor;
for use in a treatment method for promoting healing and epithelialization of a wound on the skin of a subject in need thereof, or in a method of treatment of a hair-loss disorder in a subject in need thereof; wherein said treatment method is administered subsequently or simultaneously to the irradiation in the treatment site of said subject with a source of light such as a source of red light.

2. The composition for use according to claim 1, wherein said treatment method is administered subsequently or simultaneously to the administration in the treatment site of said subject, of a photodynamic therapy comprising photosensitive agents capable of producing reactive oxygen species (ROS), such as 5-aminolevulinic acid (ALA) or its methylated derivative methyl aminolevulinate (mALA) and the irradiation with a source of light such as a source of red light.

3. The composition for use according to any of claims 1 to 2, wherein the Eng function inhibitor (iEng) is selected from the list consisting of:
a. an endoglin binding agent such as an antibody or an aptamer;
b. a short interfering RNA or silencing RNA (siRNA); and
c. the soluble extracellular domain of the human Eng protein.

4. The composition for use according to claim 3, wherein the Eng function inhibitor (iEng) is selected from the list consisting of:
a. an endoglin binding agent selected from the list consisting of SEQ ID NO 3 or SEQ ID NO 4;
b. a short interfering RNA or silencing RNA (siRNA) selected from the list consisting of SEQ ID NO 2; and
c. the soluble extracellular domain of the human Eng protein selected from the list consisting of SEQ ID NO 5.

5. The composition for use according to any of claims 1 to 2, wherein the Eng function inhibitor (iEng) is an endoglin binding agent.

6. The composition for use according to claim 5, wherein the Eng function inhibitor is the aptamer of SEQ ID NO 3 or SEQ ID NO 4 or an aptamer that, when optimally aligned with any of SEQ ID NO 3 or SEQ ID NO 4, shares at least 80% sequence identity with any of SEQ ID NO 2 or SEQ ID NO 3 and is capable inhibiting the Eng function by reducing the gene expression level of the two key target genes Id1 and Id2 of Eng signalling in skin cells in at least 80%, as measure by a gene expression quantification technique such as RT-qPCR, in comparison to said gene expression level of the same the cells not exposed to Eng function inhibitor.

7. The composition for use according to claim 5, wherein the Eng function inhibitor is the aptamer consisting of SEQ ID NO 3 or consisting of SEQ ID NO 4.

8. The composition for use according to any of claims 1 to 2, wherein the Eng function inhibitor (iEng) is the short interfering RNA or silencing RNA (siRNA) of SEQ ID NO 2 or a siRNA that, when optimally aligned with SEQ ID NO 2, shares at least 80% sequence identity with SEQ ID NO 2 and is capable of inhibiting the Eng function by reducing the gene expression level of the two key target genes Id1 and Id2 of Eng signalling in skin cells in at least 80%, as measure by a gene expression quantification technique such as RT-qPCR, in comparison to said gene expression level of the same the cells not exposed to Eng function inhibitor.

9. The composition for use according to any of claims 1 to 2, wherein the Eng function inhibitor (iEng) is the short interfering RNA or silencing RNA (siRNA) consisting of SEQ ID NO 2

10. The composition for use according to any of claims 1 to 2, wherein the Eng function inhibitor (iEng) is the soluble extracellular domain of the human Eng protein of SEQ ID NO 5 or a peptide that, when optimally aligned with SEQ ID NO 5, shares at least 80% sequence identity with SEQ ID NO 5 and is capable inhibiting the Eng function by reducing the gene expression level of the two key target genes Id1 and Id2 of Eng signalling in skin cells in at least 80%, as measure by a gene expression quantification technique such as RT-qPCR, in comparison to said gene expression level of the same the cells not exposed to Eng function inhibitor.

11. The composition for use according to any of claims 1 to 2, wherein the Eng function inhibitor (iEng) is the soluble extracellular domain of the human Eng protein consisting of SEQ ID NO 5.

12. The composition for use according to any of claims 1 to 11, wherein said use is for the treatment of a hair-loss disorder in a subject in need thereof.

13. The composition for use according to any of claims 1 to 11, wherein said use is for a treatment method for promoting healing and epithelialization of a wound on the skin of a subject in need thereof.

14. The composition for use according to claim 13, wherein the wound on the skin is a skin burn such as a sunburn.

15. The composition for use according to any of claims 1 to 14, wherein the photodynamic treatment consists of ALA or MAL and a source of light such as a source of red light.
